(19) Europäisches Patentamt
European Patent Office
Office européen des brevets

(11) Veröffentlichungsnummer : **0 091 022**
**B1**

(12) # EUROPÄISCHE PATENTSCHRIFT

(45) Veröffentlichungstag der Patentschrift : **21.11.85**

(51) Int. Cl.[4] : **C 07 D261/08**, C 07 D261/04, C 07 D261/18

(21) Anmeldenummer : **83102919.4**

(22) Anmeldetag : **24.03.83**

(54) **5-Trichlormethylisoxazol, ein Verfahren zu seiner Herstellung und seine Verwendung.**

(30) Priorität : **01.04.82 DE 3212137**
**01.04.82 DE 3212136**
**21.05.82 DE 3219049**

(43) Veröffentlichungstag der Anmeldung : **12.10.83 Patentblatt 83/41**

(45) Bekanntmachung des Hinweises auf die Patenterteilung : **21.11.85 Patentblatt 85/47**

(84) Benannte Vertragsstaaten : **AT BE CH DE FR GB IT LI SE**

(56) Entgegenhaltungen :
**FR-A- 2 230 644**
**IZVESTIYA AKADEMII NAUK, SSR, SERIYA KHIMICHESKAYA, Nr. 9, 2018 (1971)**
**JOURNAL OF AMERICAN CHEMICAL SOCIETY, 76, 5147 (1954)**
**ADVANCES IN HETEROCYCLIC CHEMISTRY, 2, 398 (1963)**

(73) Patentinhaber : **BASF Aktiengesellschaft**
**Carl-Bosch-Strasse 38**
**D-6700 Ludwigshafen (DE)**

(72) Erfinder : **Spiegler, Wolfgang, Dr.**
**Amsterdamer Strasse 16**
**D-6700 Ludwigshafen (DE)**
Erfinder : **Goetz, Norbert, Dr.**
**Schoefferstrasse 25**
**D-6520 Worms 1 (DE)**

**EP 0 091 022 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 5-Trichlormethylisoxazol der Formel I

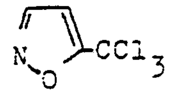

(I)

ein Verfahren zu seiner Herstellung aus 5-Trichlormethyl-5-hydroxy-2-isoxazolin (II) mit Hilfe von Dehydratisierungskatalysatoren und seine Verwendung in einem Kreisprozeß.

Es ist bekannt, daß 5-Hydroxy-2-isoxazoline zu Isoxazolen dehydratisiert werden können (The Chemistry of Heterocyclic Compounds *17*, Seiten 32 bis 105 (1962). Die Wasserabspaltung aus substituierten 5-Hydroxy-2-isoxazolinen stößt jedoch auf Schwierigkeiten. So ist bisher angenommen worden, daß Verbindungen von der Art des 5-Trichlormethylisoxazols (I) nicht durch Wasserabspaltung aus entsprechenden 5-Hydroxy-2-isoxazolinen (II) erhalten werden (Izvestiya Akademii Nauk, SSR, Seriya Khimicheskaya, Nr. 9, 2018 bzw. 1903, engl. Aus. (1971).

Der Grund dafür soll einerseits in der geringen Reaktivität einer Hydroxylgruppe in Nachbarstellung zu einem Perhalogenalkylrest liegen, die im allgemeinen eine Dehydratisierung verhindert (vgl. vorher und auch Journal of American Chemical Society *76*, 5147 (1954), zum anderen in der geringen Stabilität des Isoxazolinringes z. B. bei höheren Temperaturen oder unter basischen Reaktionsbedingungen.

5-Trichlormethylisoxazol war somit bisher mangels eines geeigneten Herstellverfahrens nicht bekannt.

Es wurde nun gefunden, daß die vorstehenden Annahmen auf einem Vorurteil beruhen, daß vielmehr 5-Trichlormethylisoxazol (I) unter bestimmten Bedingungen sehr wohl durch Dehydratisierung aus (II) erhalten werden kann.

Erfindungsgemäß läßt man auf 5-Hydroxy-5-trichlormethyl-2-isoxazolin (II) unter schonenden Bedingungen eine starke Säure bzw. deren Äquivalente einwirken. Man kann (II) hierzu entweder mit einem Überschuß der Säure bei relativ niedriger Temperatur stehen lassen oder mit katalytischen Mengen einer Säure eines sauren Salzes eines Metalloxids, oder einer Lewissäure, insbesondere Säuren mit wasserentziehender Wirkung erwärmen ; das gebildete 5-Trichlormethyl-isoxazol (I) läßt sich zusammen mit dem freigesetzten Wasser während oder nach der Reaktion aus dem Reaktionsgemisch abdestillieren.

Als Säure kommt eine starke Mineralsäure in Frage wie Schwefelsäure, Phosphorsäure, konzentrierte Salzsäure oder ggf. deren Anhydride oder starke Carbonsäuren wie z. B. Trichloressigsäure oder eine Sulfonsäure wie p-Toluolsulfonsäure.

Als saures Salz wird ein Hydrogensalz einer mehrbasischen starken Mineralsäure verwendet, wie z. B. ein Hydrogensulfat oder ein Mono- oder Dihydrogenphosphat oder ein Polyphosphat, das noch wenigstens teilweise protoniert ist.

Als Lewissäuren sind Chloride und Bromide der II., VII., VIII. Nebengruppe sowie der III. und IV. Hauptgruppe des Periodensystems geeignet, wie z. B. Eisentrichlorid, Bortrifluorid, Antimonpentachlorid usw.

Mineralsäuren mit wasserentziehender Wirkung sind insbesondere Schwefelsäure oder Polyphosphorsäure.

Die Umsetzung wird bei einer möglichst niedrigen Temperatur vorgenommen, die aber im Einzelfall bis 150 °C, oder mehr betragen kann. Die Umsetzung kann kontinuierlich (fortlaufend) oder diskontinuierlich (absatzweise) gestaltet werden.

5-Trichlormethylisoxazol (I) kann aus dem Reaktionsgemisch entweder durch Destillation oder — gegebenenfalls nach Verdünnen mit Wasser — durch Extraktion mit einem organischen Lösungsmittel isoliert und durch eine fraktionierte Destillation gereinigt werden.

Das bisher zwar postulierte, jedoch nicht zugängliche 5-Trichlormethylisoxazol (I) ist ein wichtiges Zwischenprodukt bei der Herstellung von 5-Isoxazolcarbonsäure oder 5-Isoxazolcarbonsäurechlorid (III), aus denen z. B. fungizid wirksame 5-Isoxazolcarbonsäureanilide erhalten werden (vgl. DE-OSen 29 40 189, 29 48 704).

Isoxazolcarbonsäure läßt sich aus (I) durch Hydrolyse gewinnen, wobei die erfindungsgemäße Dehydratisierung und die Hydrolyse unter gewissen Bedingungen auch gleichzeitig verlaufen, da in beiden Fällen eine Katalyse durch Säuren wirksam ist.

Hierzu ist im einzelnen folgendes zu sagen :

Zur Herstellung von 5-Isoxazolcarbonsäure sind verschiedene Methoden bekannt (Übersichtsartikel : Heterocyclic Compounds, Nr. 17, Seite 82, *1962* ; Advanced Heterocyclic Chemistry, Band *2*, Seite 365, *1963* und Band *25*, Seite 148, *1979*).

Die Seitenkettenoxidation 5-substituierter Isoxazole, wie z. B. von ungesättigten Alkylresten (Gazzetta Chimica Italiana *72*, 458, *1942*, Journal of Heterocyclic Chemistry *1968*, 621), alpha-Hydroxyalkylresten (Gazzetta Chimica Italiana *79*, 654, *1949* und *70*, 779, *1940*) sowie von 5-Formylisoxazol (Gazzetta Chimica Italiana *72*, 155, *1942*) gelingt zwar in guten Ausbeuten, doch sind die dafür benötigten

Ausgangsverbindungen nur schwer zugänglich und speziell für eine technische Synthese nicht von Interesse. Dies gilt insbesondere für in 3-Position unsubstituierte Isoxazole, da beta-Ketoaldehyde mit Hydroxylamin stets Isomerengemische der entsprechenden 3- und 5-substituierten Isoxazole ergeben (Heterocyclic Compounds, Nr. 17, Seite 10, *1962*). Auch die gängige 1,3-dipolare Cycloaddition von Nitriloxiden an Olefine oder Acetylene ist zur Herstellung von unsubstituierten 5-Isoxazolcarbonsäurederivaten wegen der Instabilität der benötigten Knallsäure für technische Verfahren ungeeignet (Chemische Berichte *106*, Seite 3291, 1973).

Die Erfindung, die 5-Trichlormethylisoxazol (I) zur Verfügung stellt, erlaubt, 5-Isoxazolcarbonsäure in hoher Ausbeute durch Hydrolyse von 5-Trichlormethylisoxazol (I) oder unmittelbar durch Dehydratisierung und Hydrolyse aus 5-Trichlormethyl-5-hydroxy-2-isoxazolin (II) zu erhalten. Sie führt somit auf einem einfacheren und wirtschaftlicheren Weg als die bisher bekannten zur 5-Isoxazolcarbonsäure.

Zwar ist die Hydrolyse der Trichlormethylgruppe als allgemeine Methode zur Herstellung von Carbonsäuren bekannt (Houben-Weyl ; Methoden der Organischen Chemie, Band 8, Seite 426), doch ist überraschend, daß sie in Gegenwart eines Isoxazolringes durchgeführt werden kann, da der Isoxazolring unter Hydrolysebedingungen leicht unter Ringöffnung gespalten wird (Advances in Heterocyclic Chemistry *2*, Seite 398). Es war auch nicht zu erwarten, daß aus II in einem Reaktionsschritt Wasser abgespalten und die Trichlormethylgruppe verseift werden kann, da schon die Dehydratisierung von II als nicht ausführbar gilt.

Als Säuren sind für die Hydrolyse von I und die gleichzeitige Dehydratisierung und Hydrolyse von II Mineralsäuren wie z. B. Schwefelsäure, Phosphorsäure, konzentrierte Salzsäure oder Lewissäuren wie z. B. Eisentrichlorid, Bortrifluorid, Antimonpentachlorid oder Titantetrachlorid geeignet. Die Umsetzung wird zweckmäßig bei einer Temperatur von 50 °C bis 250 °C, vorzugsweise von 80 °C bis 150 °C, drucklos oder unter Druck, fortlaufend oder absatzweise durchgeführt. In manchen Fällen ist eine allmähliche Steigerung der Temperatur im Reaktionsgemisch anzuraten. Nach dem Ablauf der Reaktion, die i. a. je nach Katalysator und Temperatur nicht mehr als einige Stunden dauert, wird das Reaktionsgemisch mit Wasser verdünnt und die Carbonsäure mit einem organischen Lösungsmittel extrahiert. Nach Trocknung der organischen Phase und dem Abdestillieren des Lösungsmittels fällt sie in reiner Form an.

Außerdem kann 5-Trichlormethylisoxazol (I) vorteilhaft eingesetzt werden in einem Verfahren zur Herstellung von 5-Isoxazolcarbonsäurechlorid (III), wobei auch Trichloracetylchlorid (IV) gewonnen wird und das nach fachmännischer Vorstellung der nachstehenden Reaktionsgleichung entspricht :

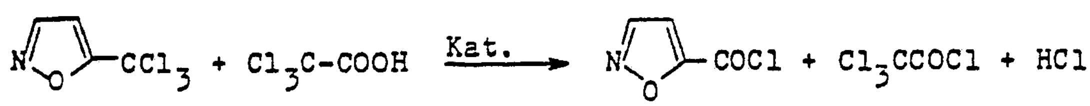


(I) (III) (IV)

5-Isoxazolcarbonsäurechlorid (III), das zur Herstellung von Fungiziden nach der US-PS 4 001 237 bzw. den DE-OSen 29 40 189 und 29 48 704 verwendet werden kann, ist bisher schwer zugänglich. Zwar liegt es nahe, es in herkömmlicher Weise aus 5-Isoxazolcarbonsäure mit Chlorierungsmitteln wie Thionylchlorid herzustellen, was tatsächlich mit hoher Ausbeute gelingt. 5-Isoxazolcarbonsäure ist aber bisher — wie erwähnt — schwer zugänglich, so daß ein solches Verfahren nicht in technischem Maßstab durchführbar war.

Nun ist, wie vorstehend angegeben, 5-Trichlormethylisoxazol (I) dadurch leicht erhältlich, daß man 5-Trichlormethyl-5-hydroxy-2-isoxazolin (II) in Gegenwart einer starken Säure oder einer Lewis-Säure erwärmt.

Allerdings läßt sich 5-Trichlormethylisoxazol nicht wie Benzotrichlorid z. B. mit Essigsäure in Gegenwart von Schwefelsäure umsetzen (vgl. US-PS 1 965 556). Unter den in der US-PS genannten Bedingungen findet nämlich überhaupt keine Reaktion statt ; vielmehr läßt sich (I) unzersetzt aus dem Reaktionsgemisch wieder abdestillieren.

Es wurde nun gefunden, daß III aus I in guter Ausbeute erhalten wird, wenn man Trichloressigsäure anstelle von Essigsäure verwendet.

Daneben wird in entsprechender Menge Trichloracetylchlorid (IV) erhalten.

Das erfindungsgemäße Verfahren ermöglicht somit einerseits die unmittelbare Herstellung von III aus I, ohne daß 5-Isoxazolcarbonsäure als Zwischenprodukt anfällt, die in einem zusätzlichen Reaktionsschritt, z. B. mit Thionylchlorid in III überführt werden müßte.

Ferner wird wie erwähnt Trichloracetylchlorid (IV) erhalten, das seinerseits als Ausgangsstoff bei der Herstellung von III zusammen mit Vinylether und Hydroxylamin verwendet werden kann. Die Gewinnung von (IV) ist sehr einfach, da sowohl (III) als auch (IV) getrennt aus dem Reaktionsgemisch abdestilliert werden können.

Das erfindungsgemäße Verfahren ist somit nicht nur im Vergleich zu bisher infrage kommenden Verfahren zur Herstellung von III einfacher und wirtschaftlicher, sondern ermöglicht darüber hinaus, einen Kreisprozeß, in den letztlich nur Vinylether, Hydroxylamin und Trichloressigsäure eingehen.

Als saure Katalysatoren können starke Protonensäuren oder Lewissäuren verwendet werden. Als Protonensäuren sind wasserfreie Mineralsäuren wie Schwefelsäure oder Sulfonsäuren wie Trifluormethansulfonsäure geeignet. Als Lewissäuren sind Chloride und Bromide der II., VII. und VIII.

Nebengruppe sowie der III. und V. Hauptgruppe des Periodensystems geeignet, wie Zink(II)-chlorid, Eisen(III) chlorid oder Antimon(V)-chlorid.

Die Umsetzung wird zweckmäßig bei einer Temperatur von 70 bis 180 °C durchgeführt, vorzugsweise bei 90 bis 140 °C ; sie läuft bei atmosphärischem Druck ab und kann kontinuierlich oder absatzweise vorgenommen werden. Je nach Bedingungen sind z. B. zwischen 2 und 12 Stunden erforderlich.

Der Verlauf der Reaktion kann an der Entwicklung von Chlorwasserstoffgas beobachtet und verfolgt werden. Trichloracetylchlorid (IV) oder ein Gemisch aus (IV) und (III) können während oder nach Beendigung der Reaktion aus dem Reaktionsgemisch abdestilliert werden. In diesem Falle ist die Anwendung von leicht vermindertem Druck günstig. Zur Reindarstellung von (III) und (IV) wird das Destillat gegebenenfalls erneut fraktioniert.

Die Verfügbarkeit von Trichloracetylchlorid ermöglicht nun die Herstellung von 5-Trichlormethyl-5-hydroxy-2-isoxazolin (II)

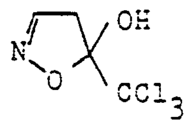

(II)

durch Anlagerung von Trichloracetylchlorid an einen Vinylether und Umsetzung des Anlagerungsproduktes mit Hydroxylamin.

Als Vinylether im Sinne der Erfindung sind Verbindungen der allgemeinen Struktur

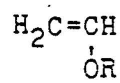

(V)

anzusehen, worin R einen unverzweigten oder verzweigten Alkylrest oder einen Arylrest bedeutet. Dem Anlagerungsprodukt (IIa) kann man je nach Reaktionsbedingungen die allgemeine Struktur

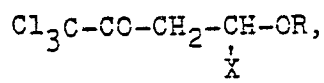

(IIa)

worin R die oben genannte Bedeutung hat und X je nach Reaktionsbedingungen ein Chloratom, eine Hydroxy- oder Alkoxy- bzw. Aryloxygruppe sein kann, oder die allgemeine Struktur

$$Cl_3C-CO-CH=CH-Y \qquad (IIb)$$

zuschreiben, worin Y je nach Reaktionsbedingungen dem vorstehenden Substituenten X (außer Chlor) entspricht, eventuell auch ein Rest eines zur Reaktionsleitung zugesetzten Amins, wie Pyrrolidin, Piperidin oder Morpholin sein kann.

Es ist bekannt, daß man (II) durch Umsetzung von 1-Diethylamino-4,4,4-trichlorbuten-3-on mit Hydroxylamin erhält (Izvestiya Akademii Nauk SSSR, Seriya Khimicheskaya, Nr. 9, Seite 2018, 1971). Nachteilig an diesem Verfahren ist, daß 1-Diethylamino-4,4,4-trichlorbuten-3-on nur in sehr schlechter Ausbeute aus Trichloracetylchlorid und Triethylamin zugänglich ist (Izvestiya Akademii Nauk SSR, Seriya Khimecheskaya, Nr. 1, Seite 112, 1969). Die dort angegebene Ausbeute beträgt 36 %, bezogen auf Trichloracetylchlorid bzw. 25 % bezogen auf Triethylamin. Zudem fällt (II) dabei stark verunreinigt an, denn es mußte zur Reinigung umkristallisiert werden.

Es ist ferner bekannt, daß Trichloracetanhydrid (Chemistry Letters, *1976*, Seite 499) oder Trichloracetylchlorid (S. Patai, The Chemistry of Functional Groups, Supplement E, The Chemistry of Ethers, Crownethers, Hydroxyl Groups and their Sulphur Analogues, Part 2, 1980, Seite 786) an Ethylvinylether addiert werden können.

Die Teilaufgabe der Erfindung, 5-Trichlormethyl-5-hydroxy-2-isoxazolin (II) herzustellen, wurde dadurch vorteilhafter als auf dem bisher bekannten Weg gelöst, daß man es aus Trichloracetylchlorid, einem Alkyl- oder Arylvinylether und Hydroxylamin herstellt.

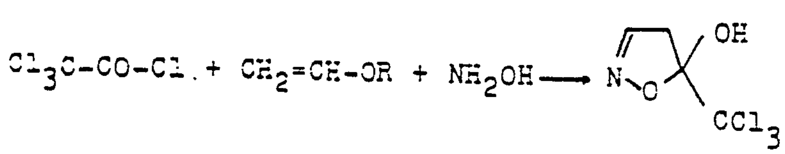

(II)

Im Vergleich zu den bisher zur Verfügung stehenden Verfahren ergibt das erfindungsgemäße Verfahren bessere Ausbeuten an (II) bezogen auf Trichloracetylchlorid und die Umsetzung verläuft rasch und ohne jede Komplikation, gegebenenfalls sogar ohne Isolierung des Anlagerungsproduktes IIa bzw. IIb. Die Verbindung II fällt dabei in reiner Form an. Das erfindungsgemäße Verfahren ist somit einfacher und wirtschaftlicher.

Als Alkyl- bzw. Arylvinylether sind solche mit Alkylgruppen von z. B. bis zu 18 C-Atomen, die auch verzweigt sein können und phenylsubstituierte Vinylether verwendbar. Da der Substituent letzlich wieder verloren geht, ist es am günstigsten, Methyl- oder Ethylvinylether zu verwenden, wenn solcher verfügbar ist.

Man verfährt zweckmäßig so, daß man zunächst Trichloracetylchlorid (IV) mit dem Vinylether (V) umsetzt. Dabei kann entweder IV zu V oder umgekehrt V zu IV zugesetzt werden. Die Reaktion verläuft mit oder bevorzugt ohne Zugabe von Lösungsmitteln und im wesentlichen stöchiometrisch.

In Frage kämen als Lösungsmittel Benzol, Toluol, Ethylbenzol, o-, m-, p-Xylol, chlorierte Kohlenwasserstoffe, z. B. Dichlormethan, Trichlormethan, Tetrachlormethan, 1,2-Dichlorethan, 1,1,1-Trichlorethan, 1,1-Dichlorpropan, Chlorbenzol, o-, m-, p-Dichlorbenzol, Nitrokohlenwasserstoffe, z. B. Nitrobenzol, o-Nitrotoluol, o-, m-, p-Chlornitrobenzol, Dialkylether, z. B. Diethylether, Diisopropylether, tert.-Butylmethylether, cyclische Ether, z. B. Tetrahydrofuran, Dioxan oder deren Gemische.

Die Umsetzung (Anlagerung) von IV und V verläuft an sich spontan ; in Gegenwart einer Base, z. B. eines Trialkylamins, eines N,N-Dialkylanilins, Pyridin oder wäßrigen Lösungen von Alkalimetall oder Erdalkalimetallhydroxyden werden eine gewisse Beschleunigung und auch weniger Nebenprodukte beobachtet ; wenn ein sekundäres Amin verwendet wird, so kann es in das Anlagerungsprodukt eingebaut werden (vgl. oben). Die Base kann entweder mit IV oder V vorgelegt bzw. zugegeben oder zum Gemisch aus IV und V zugegeben werden.

Es kann auch nach der Reaktion von IV mit V und vor Zugabe des Hydroxylamins eine Base zugegeben werden. Dies kann entweder eine der genannten Basen sein oder andere wie Metallhydroxyde, primäre oder sekundäre Amine usw. Die Basen können ohne Lösungsmittel, in einem der genannten Lösungsmittel oder in anderen wie Wasser oder Alkoholen zugegeben werden.

Die Umsetzung mit Hydroxylamin wird zweckmäßig so durchgeführt, daß man eine Lösung z. B. in Wasser, einem Alkohol oder einem Gemisch aus Wasser und Alkohol zum Anlagerungsprodukt von IV und V zugibt. Hydroxylamin kann dabei als Salz, wie z. B. als Hydrogenchlorid oder Hydrogensulfat eingesetzt, oder aus dem Salz durch Zugabe einer Base freigesetzt werden.

Das Umsetzungsprodukt (Anlagerungsprodukt) von IV und V kann als Zwischenprodukt vor der Umsetzung mit Hydroxylamin isoliert werden ; Vorteile bietet diese zusätzliche Maßnahme aber nicht.

Die beiden Teilschritte des Verfahrens verlaufen schon bei unter 0 °C und benötigen i. a. keine Temperatur oberhalb von etwa 100 °C ; sie sind aber auch gegen höhere Temperatur nicht sehr empfindlich. Bei Bedarf kann das Verfahren fortlaufend gestaltet werden. Bei Raumtemperatur beträgt die Verweilzeit in jedem Verfahrensschritt i. a. nicht mehr als etwa 10 Stunden. Durch Verwendung von Basen und/oder höhere Temperatur ist eine Beschleunigung des Umsatzes möglich. Danach fällt das Produkt II je nach dem verwendeten Lösungsmittel in fester, d. h. unlöslicher Form oder als Lösung an und kann abgesaugt oder durch Einengen des Lösungsmittels gewonnen werden.

Zusammenfassend läßt sich die Erfindung und ihre erfinderischen Teilschritte wie folgt darstellen, wobei die abkürzende Bezifferung verwendet wird, die schon bisher eingeführt wurde. Die als zweistufig angegebene Umsetzung von II über nach III kann grundsätzlich auch in einem Zug vorgenommen werden.

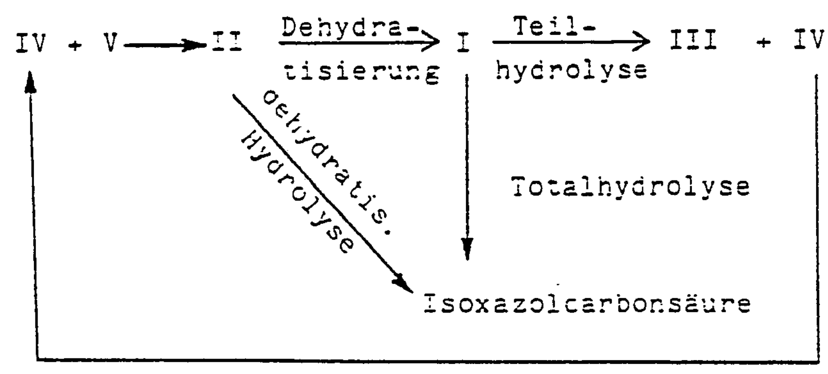


Beispiele 1 bis 9 betreffen die Herstellung von 5-Trichlormethyl-5-hydroxy-2-isoxazolin (II)

## Beispiel 1

Zu 850 g Trichloracetylchlorid werden unter Rühren bei 5 bis 10 °C 336,3 g Ethylvinylether allmählich zugesetzt und 10 Stunden bei 10 bis 25 °C nachgerührt. Dann gibt man zunächst 215 g Ethanol und anschließend eine Lösung von 324,6 g Hydroxylaminhydrochlorid in 500 ml Wasser zu und rührt über

Nacht nach. Nach Zugabe von 500 ml Wasser wird der Feststoff abgesaugt, mit wenig Toluol nachgewaschen und im Vakuum bei 50 °C getrocknet. Die Ausbeute an (II) betrug 715 g (75 % der rechnerisch möglichen). Schmp. 143-144 °C

$^1$H-NMR ($DMSO-D_6$) : δ 3,22 und 3,62 ($—CH_2—$, AB, J 19,6 Hz) ; 7,42 (N = CH, S) ; 8,22 (OH, S)

IR (KBr) : 3143, 3083, 1624, 1297, 1226, 906, 846, 831, 798

## Beispiel 2

Zu 72 g Ethylvinylether setzt man bei 5 bis 10 °C unter Rühren 182 g Trichloracetylchlorid zu und rührt 10 Stunden bei 10 bis 25 °C nach. Dann gibt man 46 g Ethanol zu und anschließend 69,5 g Hydroxylaminhydrochlorid, in 125 ml Wasser gelöst und verfährt weiter wie in Beispiel 1 beschrieben.

Es wurde (II) in einer Ausbeute von 133 g (65 %) erhalten.

## Beispiel 3

Zu 91 g Trichloracetylchlorid werden unter Rühren bei 5 bis 10 °C 36 g Ethylvinylether allmählich zugesetzt und 10 Stunden bei 10 bis 25 °C nachgerührt. Dann wird eine Lösung von 41 g Hydroxylammoniumsulfat und 42 g Natriumhydrogencarbonat in 200 ml Wasser zugesetzt, über Nacht nachgerührt, der Feststoff abgesaugt, mit Toluol gewaschen und getrocknet.

Es wurde (II) in einer Ausbeute von 82,5 g (81 %) erhalten.

## Beispiel 4

Zu 18,2 g Trichloracetylchlorid tropft man unter Rühren bei 10 bis 20 °C ein Gemisch aus 7,2 g Ethylvinylether und 7,9 g Pyridin und rührt 4 Stunden bei Raumtemperatur nach. Dann wird eine Lösung von 6,95 g Hydroxylaminhydrochlorid in 10 ml Wasser zugetropft und über Nacht nachgerührt. Nach Zugabe von 50 ml Wasser wird der Feststoff abgesaugt, mit wenig Toluol gewaschen und getrocknet.

Es wurde (II) in einer Ausbeute von 18,8 g (92 %) erhalten.

## Beispiel 5

Zu einer Lösung von 18,2 g Trichloracetylchlorid in 20 ml tert.-Butylmethylether tropft man aus einem mit Trockeneis gekühlten Tropftrichter bei 0 °C unter Rühren 5,8 g Methylvinylether und rührt 24 Stunden bei 0 bis 25 °C nach. Dann wird eine Lösung von 6,95 g Hydroxylaminhydrochlorid in 10 ml Wasser zugegeben und über Nacht gerührt. Nach Zugabe von 50 ml Toluol wird der Feststoff abgesaugt, mit Toluol gewaschen und getrocknet.

Es wurde (II) in einer Ausbeute von 13,9 g (68 %) erhalten.

## Beispiel 6

Zu einer Lösung von 18,2 g Trichloracetylchlorid in 20 ml Toluol tropft man bei 25 °C unter Rühren 29,6 g Oktadecylvinylether und rührt 24 Stunden nach. Dann wird eine Lösung von 8,2 g Hydroxylammoniumsulfat in 10 ml Wasser zugegeben, über Nacht gerührt, der Feststoff abgesaugt, mit Toluol gewaschen und getrocknet.

Es wurde (II) in einer Ausbeute von 12,3 g (60 %) erhalten.

## Beispiel 7

Zu 18,2 g Trichloracetylchlorid tropft man bei 60 °C unter Rühren 10,0 g Isobutylvinylether und rührt 3 Stunden bei 60 °C nach. Dann wird eine Lösung von 6,96 g Hydroxylaminhydrochlorid in 10 ml Wasser zugetropft und 3 Stunden bei 60 °C nachgerührt. Nach dem Abkühlen des Reaktionsgemisches wird der Feststoff abgesaugt, mit Toluol gewaschen und getrocknet.

Es wurde (II) in einer Ausbeute von 10,1 g (50 %) erhalten.

## Beispiel 8

Zu 18,2 g Trichloracetylchlorid wurde bei 5 bis 10 °C unter Rühren 7,2 g Ethylvinylether zugesetzt und bei 10 bis 25 °C über Nacht nachgerührt. Die Destillation des Reaktionsgemisches ergab 16,2 g (74 %) 1-Ethoxy-54,4,4-trichlor-1-buten-3-on.

Siedepunkt : 83 bis 85 °C/1 mbar.

$^1$H-NMR ($CDCl_3$) : δ 1,40 (t) ; 4,13 (q) ; 6,18 (d) ; 7,90 (d)

Die erhaltenen 16,2 g Ethoxytrichlorbutenon wurden mit 6 ml Ethanol verdünnt und mit einer Lösung von 6,95 g Hydroxylaminhydrochlorid in 10 ml Wasser versetzt, über Nacht nachgerührt, der Feststoff abgesaugt, gewaschen und getrocknet.

Ausbeute : 15,6 g (76 %) II.

## Beispiel 9

Zu 18,2 g Trichloracetylchlorid wurde bei 5 bis 10 °C unter Rühren 7,2 g Ethylvinylether zugesetzt um bei 10 bis 25 °C über Nacht nachgerührt. Dann wurde 100 ml Diethylether zugegeben und 14,2 g Pyrrolidin bei 0 °C zugegeben. Der Niederschlag wurde abgesaugt, mit 150 ml Essigester ausgerührt und filtriert. Das Filtrat wurde mit Wasser ausgeschüttelt, über $Na_2SO_4$ getrocknet, eingeengt und aus Petrolether umkristallisiert.

Ausbeute : 14,4 g (59 %) 1-Pyrrolidino-4,4,4-trichlor-1-buten-3-on

Fp. 103-104 °C

$^1$H-NMR ($CDCl_3$) :

7,29 g Pyrrolidinotrichlorbutenon und 2,09 g Hydroxylaminhydrochlorid wurden in 40 ml Methanol 8 Stunden auf Rückflußtemperatur erwärmt.

Das Reaktionsgemisch wurde mit 200 ml Wasser verdünnt und mit Ether extrahiert. Die Etherphase wurde über $Na_2SO_4$ getrocknet und im Rotationsverdampfer zur Trockene gebracht. Der Rückstand wurde aus Toluol/Cyclohexan umkristallisiert.

Ausbeute : 6,13 g (85 %) II.

Beispiele 10, 11 und 12 betreffen die Herstellung von Trichlormethylisoxazol (I).

## Beispiel 10

714 g 5-Trichlormethyl-5-hydroxy-2-isoxazolin (II) wurden in 700 ml 96 %iger Schwefelsäure gelöst. Nach 4-stündigem Rühren bei Raumtemperatur wurde in 5 Liter Eiswasser gegossen und mit Methylenchlorid extrahiert. Die organische Phase wurde über $Na_2SO_4$ getrocknet und das Lösungsmittel im Vakuum abdestilliert. Fraktionierung des Rückstands unter vermindertem Druck ergab 592 g (91 %) (I) mit einem Siedepunkt von 98 °C/40 mbar. Schmp. 24 bis 26 °C.

$^1$H-NMR ($CDCl_3$) : δ 6,60 (d) ; 8,24 (d), J

IR (fl. Kap.) : 3130, 1582, 1460, 1332, 1230, 1188, 1030, 1005, 918, 900, 815, 788, 765, 660, 618.

## Beispiel 11

51,1 g (II) und 0,5 g 96 %ige Schwefelsäure wurden auf 120 °C erwärmt. Während der Umsetzung wurde bei mäßig vermindertem Druck (I) zusammen mit dem gebildeten Wasser abdestilliert. Das Destillat wurde in Methylenchlorid aufgenommen, die wäßrige Phase abgetrennt und die organische Phase wie vorstehend beschrieben weiter aufgearbeitet. Ausbeute : 39 g (84 %) I.

## Beispiel 12

20,5 g II und 0,48 g Eisentrichlorid wurden auf 140 °C erwärmt. Im übrigen wurde wie in Beispiel 2 verfahren. Ausbeute : 16 g (86 %) I.

Verseifung von Trichlormethylisoxazol und 5-Trichlormethyl-5-hydroxy-2-isoxazolin (II) zur Isoxazolcarbonsäure (Dehydratisierung und Hydrolyse).

A) Ein Gemisch aus 60,0 g 5-Trichlormethylisoxazol (I), 5,8 ml Wasser und 20 ml 96 %ige Schwefelsäure wurden 8 Stunden auf 100 °C erwärmt. Nach dem Abkühlen wurde in 600 ml Eiswasser eingerührt, mit Essigester extrahiert, die organische Phase über $Na_2SO_4$ getrocknet und das Lösungsmittel i. v. abdestilliert. Der Rückstand wurde mit Methylenchlorid gewaschen und im Vakuum getrocknet.

Ausbeute : 34,4 g (96 %) 5-Isoxazolcarbonsäure

Schmelzpunkt : 144 °C

$^1$H-NMR (DMSO-$D_6$) : δ = 7,22 (d) ; 8,88 (d)

IR (KBr) : 3140, 1745, 1733, 1590, 1486, 1438, 1350, 1292, 1250, 1215, 1150, 1143, 939, 914, 737.

B) 622 g 5-Trichlormethyl-5-hydroxy-2-isoxazolin (II) wurden in 210 ml 96 %iger Schwefelsäure gelöst, dann das Gemisch auf 100 °C erwärmt. Während 8 Stunden wurde HCl-Entwicklung beobachtet ; es wurde nach Abkühlen auf 5 l Eiswasser gegossen und wie vorstehend beschrieben aufgearbeitet.

Ausbeute : 322 g (94 %) Isoxazolcarbonsäure.

Verseifung von Trichlormethylisoxazol (Partialhydrolyse). 93 g 5-Trichlormethylisoxazol (I) und 22,6 g Eisen(III) chloridhexahydrat wurden für 2 Stunden auf 120 °C gehalten. Dabei wurde eine ständige Entwicklung von Chlorwasserstoff beobachtet. Anschließend wurde destilliert und 51 g (78 % der erwarteten Menge) 5-Isoxazolcarbonsäurechlorid (III) erhalten. Spt. 74 bis 78 °C.

Die Beispiele 13 bis 15 betreffen die Umsetzung von Trichlormethylisoxazol (I) und Trichloressigsäure unter Gewinnung von Trichloracetylchlorid und Isoxazolcarbonsäurechlorid (III).

## Beispiel 13

Zu einem Gemisch aus 93 g 5-Trichlormethylisoxazol (I) und 4,5 g Antimon (V)-chlorid werden bei

120 °C unter Rühren während 3 Stunden 82 g Trichloressigsäure aus einem auf 60 °C beheizten Tropftrichter zugetropft, dann wird noch 2 Stunden bei 120 °C gerührt. Während der Umsetzung werden insgesamt 78 g Trichloracetylchlorid (IV) unter vermindertem Druck (250 mbar) aus dem Reaktionsgemisch abdestilliert. Anschließend wird die Vorlage gewechselt, auf − 20 °C gekühlt und bei 20 mbar noch ein Gemisch aus (III) und (IV) aus dem Reaktionsgefäß abdestilliert. Durch anschließende fraktionierte Destillation werden aus diesem Gemisch 9 g (IV) und 50 g (III) (Siedepunkt 74-76 °C/20 mbar) erhalten, was insgesamt 96 % bzw. 77 % der erwarteten Menge an Trichloracetylchlorid (IV) und 5-Isoxazolcarbonsäurechlorid (III) entspricht.

Spektroskopische Daten von III :
$^1$H-NMR ($CDCl_3$) $\delta$ = 7,14 (d), 8,36 (d).

Beispiel 14

93 g 5-Trichlormethylisoxazol (I), 82 g Trichloressigsäure und 4 g Eisen(III)-chlorid werden unter Rühren für 8 Stunden bei 120 °C gehalten. Wenn die HCl-Entwicklung nachgelassen hat, wird unter vermindertem Druck ein Gemisch aus Trichloracetylchlorid und 5-Isoxazolcarbonsäurechlorid in eine auf − 20 °C gekühlte Vorlage destilliert. Die fraktionierte Destillation des Gemisches aus III und IV ergibt 48 g (72 % der erwarteten Menge) 5-Isoxazolcarbonsäurechlorid (III), 84 g (93 % der erwarteten Menge) Trichloracetylchlorid (IV).

Beispiel 15

23 g I, 20 g Trichloressigsäure und 0,3 g konz. Schwefelsäure werden 12 h bei 130 °C gehalten. Dann wird wie vorstehend beschrieben aufgearbeitet. 8,7 g 5-Isoxazolcarbonsäurechlorid (53 % der erwarteten Menge), 18 g Trichloracetylchlorid (81 % der erwarteten Menge).

Vollständige Darstellung des Kreisprozesses.

Zu 85 g des nach Beispiel 13 erhaltenen Trichloracetylchlorids (IV) wird unter Rühren bei 5 bis 10 °C ein Gemisch aus 33,6 g Ethylvinylether und 37 g Pyridin getropft und 4 Stunden bei 10 bis 25 °C gerührt. Dann tropft man eine Lösung von 32,5 g Hydroxylaminhydrochlorid in 50 ml Wasser zu und rührt über Nacht weiter. Nach Zugabe von 250 ml Wasser wird abgesaugt, mit wenig Toluol gewaschen und i. V. bei 50 °C getrocknet. Man erhält 88 g (92 % der erwarteten Menge) 5-Hydroxy-5-trichlormethyl-2-isoxazolin (Schmelzpunkt 143 bis 144 °C).

71,5 g des so erhaltenen 5-Hydroxy-5-trichlormethyl-2-isoxazolins werden in 70 ml 96 %iger Schwefelsäure gelöst. Nach 4 stündigem Rühren bei Raumtemperatur wird in 500 ml Eiswasser gegossen und mit Methylenchlorid extrahiert. Die organische Phase wird über $Na_2SO_4$ getrocknet und das Lösungsmittel i. V. abdestilliert. Der Rückstand wird i. V. fraktioniert. Man erhält 59,2 g 5-Trichlormethylisoxazol (91 % der erwarteten Menge, Siedepunkt 98 °C/40 mbar).

Das so erhaltene 5-Trichlormethylisoxazol kann, wie im Beispiel 13 beschrieben, mit Trichloressigsäure zu 5-Isoxazolcarbonsäurechlorid (III) und Trichloracetylchlorid (IV) umgesetzt werden.

**Patentansprüche**

1. 5-Trichlormethylisoxazol.

2. Verfahren zur Herstellung von 5-Trichlormethylisoxazol, dadurch gekennzeichnet, daß man 5-Trichlormethyl-5-hydroxy-2-isoxazolin in Gegenwart eines dehydratisierend wirkenden sauren Katalysators einer ausreichenden Temperatur aussetzt.

3. Verfahren nach Anspruch 2, dadurch gekennzeichnet, daß man als Katalysator Schwefelsäure, Phosphorsäure oder Polyphosphorsäure verwendet.

4. Verwendung von 5-Trichlormethylisoxazol zur Herstellung von 5-Isoxazolcarbonsäure, deren Säurehalogenid und/oder deren fungizid wirksamen Aniliden.

5. Verfahren zur Herstellung von 5-Isoxazolcarbonsäurechlorid, dadurch gekennzeichnet, daß man 5-Trichlormethylisoxazol in Gegenwart eines sauren Katalysators mit Trichloressigsäure umsetzt.

6. Verfahren nach Anspruch 5, dadurch gekennzeichnet, daß man als sauren Katalysator Schwefelsäure, Eisen(III)-chlorid, Antimon(V)-chlorid oder Zink(II)-chlorid verwendet.

7. Verfahren nach Anspruch 5, dadurch gekennzeichnet, daß man Trichloracetylchlorid gewinnt.

8. Ausübung des Verfahrens nach Anspruch 5 in einem Kreisprozeß, dadurch gekennzeichnet, daß man Trichloracetylchlorid mit einem Vinylether und Hydroxylamin zu 5-Trichlormethyl-5-hydroxy-2-isoxazolin umsetzt, dieses zu 5-Trichlormethylisoxazol dehydratisiert und gegebenenfalls das so gewonnene 5-Trichlormethylisoxazol zur Umsetzung gemäß Anspruch 5 verwendet.

9. Verfahren zur Herstellung von 5-Trichlormethyl-5-hydroxy-2-isoxazolin, dadurch gekennzeichnet, daß man Trichloracetylchlorid und einen Vinylether umsetzt und auf das Umsetzungsprodukt Hydroxylamin einwirken läßt.

10. Verfahren nach Anspruch 9, dadurch gekennzeichnet, daß man die Umsetzung oder einen ihrer Schritte in Gegenwart einer Base vornimmt.

**Claims**

1. 5-Trichloromethylisoxazole.

2. A process for the preparation of 5-trichloromethylisoxazole, wherein 5-trichloromethyl-5-hydroxy-2-isoxazoline is heated at an adequate temperature in the presence of an acidic catalyst having a dehydrating action.

3. A process as claimed in claim 2, wherein the catalyst used is sulfuric acid, phosphoric acid or polyphosphoric acid.

4. The use of 5-trichloromethylisoxazole for the preparation of 5-isoxazolecarboxylic acid, its acid halide and/or its fungicidally effective anilides.

5. A process for the preparation of 5-isoxazolecarboxylic acid chloride, wherein 5-trichloromethylisoxazole is reacted with trichloroacetic acid in the presence of an acidic catalyst.

6. A process as claimed in claim 5, wherein the acidic catalyst used is sulfuric acid, iron(III) chloride, antimony(V) chloride or zinc(II) chloride.

7. A process as claimed in claim 5, wherein trichloroacetyl chloride is obtained.

8. The operation of a process as claimed in claim 5 as a cyclic process, wherein trichloroacetyl chloride is reacted with a vinyl ether and hydroxylamine to give 5-trichloromethyl-5-hydroxy-2-isoxazoline, this is dehydrated to give 5-trichloromethylisoxazole and, if appropriate, the 5-trichloromethylisoxazole thus obtained is used for the reaction as claimed in claim 5.

9. A process for the preparation of 5-trichloromethyl-5-hydroxy-2-isoxazoline, wherein trichloroacetyl chloride is reacted with a vinyl ether, and hydroxylamine is allowed to act on the reaction product.

10. A process as claimed in claim 9, wherein the reaction or one of its steps is carried out in the presence of a base.

**Revendications**

1. Le 5-trichlorométhyl-isoxazole.

2. Procédé de préparation du 5-trichlorométhylisoxazole, caractérisé en ce que l'on chauffe de la 5-trichlorométhyl-5-hydroxy-2-isoxazoline à une température suffisamment élevée en présence d'un catalyseur acide à action déshydratante.

3. Procédé suivant la revendication 2, caractérisé en ce que l'on emploie comme catalyseur acide de l'acide sulfurique ou de l'acide phosphorique ou un acide polyphosphorique.

4. Utilisation du 5-trichlorométhyl-isoxazole pour la préparation de l'acide 5-isoxazol-carboxylique, d'halogénures de cet acide ou(et) des anilides à activité fongicide de celui-ci.

5. Procédé de préparation du chlorure de l'acide 5-isoxazol-carboxylique, caractérisé en ce que l'on fait réagir le 5-trichlorométhyl-isoxazole en présence d'un catalyseur acide avec l'acide trichlor-acétique.

6. Procédé suivant la revendication 5, caractérisé en ce que l'on emploie comme catalyseur acide l'acide sulfurique, le chlorure ferrique, le chlorure d'antimoine pentavalent ou le chlorure de zinc bivalent.

7. Procédé suivant la revendication 5, caractérisé en ce que l'on obtient du chlorure de trichlor-acétyle.

8. Mise en œuvre du procédé suivant la revendication 5 dans un procédé cyclique, caractérisé en ce que l'on fait réagir du chlorure de trichlor-acétyle avec un éther vinylique et l'hydroxyl-amine, transforme la 5-trichlorométhyl-5-hydroxy-2-isoxazoline obtenue par déshydratation en 5-trichlorométhyl-isoxazole et emploie éventuellement ce 5-trichlorométhyl-isoxazole pour la réaction suivant la revendication 5.

9. Procédé de préparation de la 5-trichlorométhyl-5-hydroxy-2-isoxazoline, caractérisé en ce que l'on fait réagir du chlorure de trichlor-acétyle avec un éther vinylique et le produit réactionnel obtenu avec l'hydroxylamine.

10. Procédé suivant la revendication 9, caractérisé en ce que la réaction ou un des stades de celle-ci est réalisé en présence d'une base.